# FASCICULE DE BREVET EUROPEEN

(11) **EP 0 893 989 B1**
(45) Date de publication et mention de la délivrance du brevet: **08.09.2004**
(21) Numéro de dépôt: 97903434.5
(22) Date de dépôt: 10.02.1997
(51) Int. Cl.: A61K 9/00

(54) **COMPOSES ASSOCIANT DES SUBSTANCES ALLOPATHIQUES ET LEURS CORRESPONDANTS HOMEOPATHIQUES**
MITTEL DAS ALLOPATHISCHE UND IHRE ENTSPRECHENDEN HOMEOPHATISCHEN SUBSTANZEN KOMBINIERT
COMPOUNDS COMBINING ALLOPATHIC SUBSTANCES WITH CORRESPONDING HOMEOPATHIC SUBSTANCES

(30) Priorité: 14.02.1996 FR 9602042
(43) Date de publication de la demande: 03.02.1999
(73) Titulaire: Castel, Hervé, 13650 Meyrargues (FR)
(72) Inventeur: Castel, Hervé, 13650 Meyrargues (FR)
(74) Mandataire: Roman, Michel
(86) Numéro de dépôt international: PCT/FR1997/000259
(87) Numéro de publication internationale: WO 1997/029737

(56) Documents cités:
- DE-A- 3 829 442
- FR-A- 2 660 191

## Description

La présente invention a pour objet des composés associant des substances allopathiques et leurs correspondants homéopathiques.

Elle concerne le domaine industriel de la fabrication et de la diffusion commerciale de médicaments, de compléments alimentaires ou d'aliments enrichis destinés à l'être humain ou aux différents êtres vivants du règne végétal ou animal.

L'évolution générale de la médecine et de la thérapeutique fait que l'homéopathie a son champ d'action et ses limites, et que le problème thérapeutique est d'abord résolu par le choix du mode d'action : thérapeutique classique ou thérapeutique homéopathique, ces deux méthodes, pourtant complémentaires à bien des égards, n'étant presque jamais associées.

Toutefois des spécialités pharmaceutiques formées du mélange de substances allopathiques et homéopathiques ont été mentionnées dans plusieurs documents.
Par exemple le brevet français N° 2 618 677 fait état d'une composition pharmaceutique, caractérisée par le fait qu'elle contient dans un milieu alcoolique physiologiquement acceptable et antiseptique au moins un agent à effet phlogistique choisi parmi la poudre de Cantharide, Causticum d'Hahneman ou l'iode, à des doses homéopathiques, au moins un agent ayant un effet de stimulation des membranes cytoplasmiques eucaryotes choisi parmi les oligo-éléments ou le phosphore, à des doses homéopathiques et au moins un agent ayant un effet de tannage et d'inhibition des cores viraux et des membranes bactériennes constitué par du phénol.
Un autre brevet français, publié sous le numéro 2 684 002, décrit des compositions destinées au traitement du SIDA, constituées de solutions aqueuses contenant des dérives organosiliciés possédant des fonctions silanols, stabilisées par un ou plusieurs acides organiques. Ces compositions constituent une base, qui est potentialisée par l'ajout de dérives soufrés organiques et minéraux ou/et des teintures mères telles que celles d'insectes piqueurs et suceurs ou de leurs dilutions homéopathiques.
Cependant, à notre connaissance, il n'existe aucune mention de médicaments ou autres composés associant une substance de type allopathique avec la même substance traitée selon la voie homéopathique.

Aujourd'hui encore, un grand nombre de praticiens se méfient de l'homéopathie ou même la rejettent catégoriquement. Et pourtant ses origines remontent à la tradition hippocratique. Au début du seizième siècle, Paracelse, s'inspirant d'Hippocrate, mettra en valeur l'individualisation du malade, l'individualisation du remède et la loi de similitude, ce qui en fait le précurseur de l'homéopathie, dont Samuel Hahnemann devait être le fondateur deux cent cinquante ans plus tard.
Laennec eut la gloire de découvrir le lien entre les constatations anatomopathologiques et les signes physiopathologiques présentés par le malade, ce qui représente le fondement de la médecine dite classique. Hahnemann, vers la même époque, établissait un lien analogue entre ces mêmes signes physiopathologiques et les caractéristiques de "physiologie humaine expérimentale" développées, chez le sujet sain, par les substances médicamenteuses. Découvertes simultanées, qui se complètent admirablement, l'une organisant les symptômes pour définir les maladies, l'autre organisant les symptômes pour définir les variétés individuelles des maladies et trouver les remèdes individuels correspondant.
La dilution n'était pas à l'origine même de la doctrine homéopathique, dont le principe fondamental repose sur la loi de similitude. Néanmoins, l'expérience de Hahnemann lui montra - et tous ses disciples le vérifièrent à des degrés divers - que certaines substances agissent mieux lorsqu'elles sont administrées en petite quantité ou à dose extrêmement faible, voire à dose infinitésimale, et en particulier sous forme diluée et dynamisée, ce qui permet d'éviter l'action primitive du remède, dite d'aggravation.

La présente invention, s'appuyant sur les considérations qui précèdent, a pour but de proposer des médicaments, des compléments alimentaires ou des aliments enrichis cumulant les effets des remèdes allopathiques et ceux des préparations homéopathiques, afin d'obtenir une efficacité renforcée.

Ces composés sont obtenus en mélangeant à une substance allopathique de base, dans des proportions bien définies, la même substance diluée et dynamisée selon les méthodes homéopathiques sur un support eau, alcool-eau, alcool-glycérine-Eau, lactose, lactose-Saccharose ou lactose-Fructose-Saccharose.

La description détaillée ci-après se rapporte à des exemples non limitatifs de formes de réalisation de l'objet de l'invention.

Actuellement l'élaboration des médicaments a recours à deux techniques séparées, soit allopathique avec des substances synthétiques ou naturelles matérielles, soit homéopathique avec des substances diluées dynamisées. La présente invention consiste à regrouper ces deux techniques pour augmenter l'efficacité de ces substances, c'est-à-dire à mélanger à une substance de base, la même substance diluée et dynamisée

Les proportions du mélange seront de 20 à 90 % de substance de base pour 80 à 1 % de substance diluée dynamisée.
La dilution homéopathique se fera de préférence sur un support (véhicule) Eau, Alcool-eau, alcool-Glycérine-Eau, Lactose, Lactose-Saccharose ou Lactose-Fructose-Saccharose. Il s'agira de dilutions dynamisatrices centésimales, de dilutions dynamisatrices décimales ou de dilution dynamisatrice de type Korsakow.
Par exemple, pour le traitement du diabète, l'effet de l'insuline habituellement administrée seule, pourra être renforcé par un mélange constitué de :

| | |
|---|---|
| Insuline | 90 à 99 % |
| + Insuline homéopathique | 1 à 10 % |

Du fait même des progrès de la chimiothérapie et de l'antibiothérapie, la pathologie dans son ensemble devient beaucoup plus une pathologie de malaises, de troubles, de déséquilibres, qu'une pathologie de maladie bien définie. Dans cette perspective, l'homéopathie, dans bien des cas, s'adapte remarquablement à l'ensemble morbide pour lequel aucun diagnostic précis ne peut être établi et pour lequel aucune thérapeutique rationnelle classique ne peut être déterminée.

C'est ainsi que le complexe substance de base plus substance homéopathique est particulièrement indiqué dans le cas du complément alimentaire qui est une combinaison de vitamines, d'acides aminés, d'enzymes et de sels minéraux.

| Exemple: | |
|---|---|
| Vitamine C | 90 % |
| + Vitamine C homéopathique DH3 | 10 % |

Les autres constituants peuvent également être associés à leurs correspondants homéopathiques.
Des aliments enrichis peuvent être réalisés en y incorporant des vitamines, acides aminés, enzymes et sels minéraux associés à leurs compléments homéopathiques.

Les médicaments, compléments alimentaires ou aliments enrichis selon l'invention pourront se présenter sous toutes les formes habituellement utilisées pour les produits de même catégorie : pilules, comprimés, granulés, gélules, sirops, ampoules injectables, suppositoires, poudre en sachets, etc. De même la fabrication et le conditionnement pourront s'effectuer de la même manière que pour les médicaments, compléments alimentaires ou aliments enrichis classiques, la substance homéopathique étant incorporée au cours de ladite fabrication.

Le positionnement des divers éléments constitutifs donne à l'objet de l'invention un maximum d'effets utiles qui n'avaient pas été, à ce jour, obtenus par des produits similaires.

## Revendications

1. Composés associant des substances allopathiques et leurs correspondants homéopathiques, destinés à la fabrication de compléments alimentaires ou aliments enrichis destinés à l'être humain, ou aux différents êtres vivants du règne végétal ou animal, constitués du mélange d'une substance de base avec la même substance diluée,
**caractérisés en ce qu'**ils sont constitués de compléments alimentaires tels que vitamines, acides aminés enzymes et sels minéraux mélangés, dans des proportions bien définies, aux mêmes substances diluées et dynamisées selon les méthodes homéopathiques sur un support lactose, lactose-Saccharose ou lactose-Fructose-Saccharose.

2. Composés selon la revendication 1, **se caractérisant par le fait que** la proportion de substance homéopathique diluée dynamisée est comprise entre 1 à 80 pour cent du poids total.

3. Composés selon l'une quelconque des revendications précédentes, **se caractérisant par le fait que** la substance homéopathique est obtenue par une dilution dynamisatrice décimale de la substance de base.

4. Composés selon l'une quelconque des revendications 1 et 2 **se caractérisant par le fait que** la substance homéopathique est obtenue par une dilution dynamisatrice centésimale de la substance de base.

5. Composés selon l'une quelconque des revendications 1 et 2 **se caractérisant par le fait que** la substance homéopathique est obtenue par une dilution dynamisatrice korsakovienne.

6. Complément alimentaire à base de vitamines, minéraux, enzymes, acides aminés, **caractérisé en ce qu'**au moins l'un de ses constituants est composé du mélange d'une substance avec la même substance diluée et dynamisée suivant les techniques homéopathiques.

7. Aliments enrichis **caractérisés en ce qu'**ils comportent des vitamines, sels minéraux, enzymes et des acides aminés associés à leur correspondant homéopathiques dilués et dynamisés suivant les techniques homéopathiques.

## Patentansprüche

1. Zusammensetzungen von allopathischen Substanzen und den ihnen entsprechenden homöopathischen Substanzen zur Herstellung von Nahrungsmittelergänzungen oder angereicherten Nahrungsmitteln für Menschen oder sonstige Lebewesen des Tier- und Pflanzenreichs, bestehend aus einer Grundsubstanz, gemischt mit der gleichen Substanz in verdünnter Form, **gekennzeichnet dadurch, dass** sie aus Nahrungsmittelergänzungen bestehen, wie Vitaminen, Aminosäuren, Enzymen und Mineralsalzen, die in genau definierten Anteilen mit den gleichen Substanzen gemischt werden, nachdem diese zuvor in homöopathischen Verfahren auf Lactose-, Lactose-Saccharose- oder Lactose-Fructose-Saccharose-Basis verdünnt und potenziert wurden.

2. Zusammensetzungen nach Anspruch 1, **gekennzeichnet dadurch,**
**dass** der Anteil der homöopathisch verdünnten, potenzierten Substanz zwischen 1 % und 80 % des Gesamtgewichts liegt.

3. Zusammensetzungen nach einem der vorherigen Ansprüche, **gekennzeichnet dadurch,**
**dass** die homöopathische Substanz durch Potenzierung einer Grundsubstanz auf Dezimal-Verdünnung erhalten wird.

4. Zusammensetzungen nach einem der Ansprüche 1 oder 2, **gekennzeichnet dadurch,**
**dass** die homöopathische Substanz durch Potenzierung einer Grundsubstanz auf Centisimal-Verdünnung erhalten wird.

5. Zusammensetzungen nach einem der Ansprüche 1 oder 2, **gekennzeichnet dadurch, dass** die homöopathische Substanz durch Potenzierung einer Grundsubstanz auf Korsakov-Verdünnung erhalten wird.

6. Nahrungsmittelergänzung auf Basis von Vitaminen, Mineralien, Enzymen, Aminosäuren, **gekennzeichnet dadurch,**
**dass** mindestens einer der Bestandteile aus der Mischung einer Substanz mit der nach einem homöopathischen Verfahren verdünnten und potenzierten gleichen Substanz besteht.

7. Angereicherte Nahrungsmittel, **gekennzeichnet dadurch,**
**dass** sie Vitamine, Mineralsalze, Enzyme und Aminosäuren in Zusammensetzung mit ihren jeweiligen homöopathischen Entsprechungen enthalten, die zuvor nach homöopathischen Verfahren verdünnt und potenziert wurden.

## Claims

1. Compound associating allopathic substances and their corresponding homeopathic substances, intended for the manufacture of food supplements or enriched foods for consumption by humans, or various living beings from the plant or animal kingdom, consisting of the mixture of a basic substance with the same diluted substance,
**characterized in that** they consist of food supplements such as vitamins, amino-acid enzymes and mineral salts, in well defined proportions, mixed with the same substances diluted and dynamised according to homeopathic methods on a lactose, lactose-Saccharose or lactose-Fructose-Saccharose support.

2. Compound according to claim 1, **characterized in that** the proportion of diluted and dynamised homeopathic substance is between 1 and 80 percent of the total weight.

3. Compound according to any of the aforesaid claims, **characterized in that** the homeopathic substance is obtained by decimal dynamising dilution of the basic substance.

4. Compound according to any of claims 1 and 2 **characterized in that** the homeopathic substance is obtained by a centesimal dynamising dilution of the basic substance.

5. Compound according to any of claims 1 and 2 **characterized by** the fact that the homeopathic substance is obtained by a dynamising korsakovian dilution.

6. Food complement containing vitamins, minerals, enzymes, amino acids, **characterized in that** at least one of its components consists of the mixture of a substance with the same substance diluted and dynamised according to homeopathic techniques.

7. Enriched foodstuffs **characterized in that** they comprise vitamins, mineral salts, enzymes and homeopathic amino-acids associated with their corresponding homeopathic substances diluted and dynamised according to homeopathic techniques.
